# EUROPÄISCHE PATENTSCHRIFT

(11) **EP 1 959 839 B1**
(45) Veröffentlichungstag und Bekanntmachung des Hinweises auf die Patenterteilung: **01.09.2010**
(21) Anmeldenummer: 06792221.1
(22) Anmeldetag: 22.09.2006
(51) Int. Cl.: A61B 17/02

(54) **EINRICHTUNG ZUM ABSTÜTZEN DER BAUCHDECKE GEBENÜBER DARUNTERLIEGENDEN ORGANEN BEI DER MINIMALINVASIVEN CHIRURGIE**
DEVICE FOR SUPPORTING THE ABDOMINAL WALL IN RELATION TO UNDERLYING ORGANS IN MINIMALLY INVASIVE SURGERY
DISPOSITIF DE SOUTIEN DIAPHRAGME VIS-A-VIS DES ORGANES SITUES SOUS CELUI-CI EN CHIRURGIE MINIMALEMENT INVASIVE

(30) Priorität: 11.11.2005 DE 102005053831
(43) Veröffentlichungstag der Anmeldung: 27.08.2008
(73) Patentinhaber: Haindl, Hans, 30974 Wennigsen (DE)
(72) Erfinder: Haindl, Hans, 30974 Wennigsen (DE)
(74) Vertreter: Wagner, Carsten
(86) Internationale Anmeldenummer: PCT/EP2006/009216
(87) Internationale Veröffentlichungsnummer: WO 2007/054156

(56) Entgegenhaltungen:
- US-A1- 2002 013 601
- US-A1- 2005 182 438

## Beschreibung

Die Erfindung betrifft eine Einrichtung zum Abstützen der Bauchdecke gegenüber darunterliegenden Organen zum Freihalten eines Operationsraumes bei der minimalinvasiven Chirurgie.

Bei der minimalinvasiven Chirurgie der Bauchhöhle ist es allgemein bekannt, diese mit Kohlendioxidgas zu füllen, um so einen gasgefüllten Hohlraum zu bilden, in dem genügend Platz für das Arbeiten des Operateurs mit durch die Bauchdecke hindurchgeführten Instrumenten besteht. Dieses allgemein bekannte Verfahren hat mehrere Nachteile. Ein Nachteil besteht darin, daß der Druck in der Bauchhöhle zu einer Verschlechterung des venösen Rückflusses und damit zu hämodynamischen Effekten führt, die für den Patienten nachteilig sein können. Ein weiterer Nachteil besteht darin, daß das Kohlendioxid über das Bauchfell in nicht unerheblichem Maße resorbiert wird, wodurch es zu einer Verschiebung der Stoffwechsellage des Patienten bis hin zu einer Übersäuerung führt. Dieses Kohlendioxid muß bei der Narkose wieder abgeatmet werden, jedoch läßt sich eine Übersäuerung des Gewebes trotzdem nicht vermeiden. Die Übersäuerung des Gewebes ist außerdem der Wundheilung nicht zuträglich.

Ein weiterer Nachteil besteht darin, daß das eingeblasene Kohlendioxid zwar eine übersichtliche Gasblase im Bauchraum erzeugt, jedoch liegt diese aber nicht unbedingt an der richtigen Stelle, mit der Folge, daß das Operationsgebiet, an das der Chirurg heran möchte, nicht zugänglich ist und unter Umständen sogar durch andere Strukturen, z. B. Darmschlingen, verborgen sein kann. Da diese Darmschlingen dann mit entsprechend kleinen, durch die Bauchhöhle geführten Instrumenten beiseite gehalten werden müssen, entsteht bei der Operation zusätzlicher Personalbedarf, nur um Organe beiseite zu halten. Da beiseite gehaltene Organe, z. B. Darmschlingen, sehr empfindlich sind, besteht die Gefahr, daß es beim Beiseitehalten mit metallischen Instrumenten, z. B. Fingerretraktoren, zu Verletzungen kommen kann.

Um diese Nachteile zu vermeiden, sind verschiedene Vorrichtungen bekannt, mit denen die Bauchhöhle angehoben bzw. abgestützt werden kann. Durch CA 2 109 795 ist eine Vorrichtung bekannt, bei der an Enden drehbar parallel zueinander gehaltener Stangen sich radial erstreckende Arme angeordnet sind. Diese liegen in einer Ruhestellung unmittelbar nebeneinander, so daß sie in eine Öffnung in der Bauchdecke eingeführt werden können. Innerhalb der Bauchhöhle werden diese beiden Arme fächerförmig durch Drehen der Stangen gespreizt, so daß sie die Bauchdecke untergreifen, die somit mittels der Vorrichtung angehoben werden kann. Eine ähnliche Vorrichtung ist durch US 5 820 555 bekannt. Ein Nachteil beider bekannten Vorrichtungen besteht darin, daß die Vorrichtungen nach Untergreifen der Bauchdecke ständig im angehobenen Zustand gehalten werden müssen. Dies geschieht im Falle der genannten US-Patentschrift durch ein seitlich vom Patienten angeordnetes Stativ.

Durch US 5 613 939 ist eine Einrichtung bekannt, die in gleicher Weise wirkt wie die zuvor beschriebenen bekannten Einrichtungen, von denen sie sich dadurch unterscheidet, daß die Arme durch flexible langgestreckte Hohlkörper gebildet sind, die zunächst beim Einführen durch eine Öffnung in der Bauchdecke zusammengefaltet sind und dann innerhalb der Bauchhöhle pneumatisch oder hydraulisch gefüllt werden, so daß sie steif sind und die Bauchdecke untergreifen. Die für die zuvor beschriebenen bekannten Einrichtungen angegebenen Nachteile gelten auch für diese bekannte Einrichtung in gleicher Weise.

Durch US 5 520 609 ist eine Einrichtung zum Anheben bzw. Abstützen der Bauchdecke gegenüber darunterliegenden Organen zum Zwecke der minimalinvasiven Chirurgie bekannt, die aus mehreren, über eine Schlauchleitung füllbaren, schlauchförmigen und eine Zylinderform bildenden, dünnwandigen und zusammenfaltbaren Hohlkörpern besteht. Die Hohlkörper haben dabei die Form von Ringen, die in Achsrichtung miteinander verbunden sind und jeweils an eine Schlauchleitung angeschlossen sind, über die sie nach Einführen in eine Bauchhöhle aufgeblasen werden können. Diese bekannte Einrichtung ist verhältnismäßig kompliziert, hat eine geringe seitliche Steifigkeit und erfordert eine relativ große Wandstärke, was das Einbringen durch eine kleine Öffnung in der Bauchdecke erschwert.

US 2002/0013601 A1 offenbart insbesondere eine Einrichtung zum Abstützen der Bauchdecke gegenüber darunterliegenden Organgen zum Freihalten eines Operationsraumes bei der minimalinvasiven Chirurgie gemäß dem Oberbegriff des Anspruchs 1 mit mehreren, über eine Schlauchleitung füllbaren schlauchförmigen und eine Torroidform bildenden, dünnwandigen und zusammenfaltbaren Hohlkörpern, wobei die schlauchförmigen Hohlkörper langgestreckt sind und sich in Richtung der Torroidform erstrecken, die schlauchförmigen Hohlkörper in Umfangsrichtung der Torroidform miteinander verbunden sind, wobei sich die langgestreckten Körper in Achsrichtung der Torroidform mit ihren Enden an jeweils einem schlauchförmigen, dünnwandigen, zusammenfaltbaren, einen Ring bildenden und über eine Schlauchleitung füllbaren Hohlkörper abstützen, die sich längs der beiden Ränder der Torroidform erstrecken und die Durchmesser der beiden einen Ring bildenden Hohlkörper verschieden sind, derart, daß die langgestreckten Hohlkörper eine kegelstumpfförmige Wandung bilden.

US 2005/0182438 A1 offenbar eine gleichartige Einrichtung wie die in US 2002/0013601 A1 offenbarte, jedoch ohne kegelstumpfförmige Wandung.

Nachteilig bei US 2002/0013601 A1 und US 2005/0182438 A1 ist die Tatsache, daß bei Verletzung der langgestreckten Hohlkörper die Lumen erzeugenden Vorrichtungen mehr oder weniger in sich zusammenfallen und eine weitere Operation vor Ort dann nicht mehr möglich ist.

Der Erfindung liegt die Aufgabe zugrunde, eine Einrichtung der im Oberbegriff des Anspruchs 1 genannten Art zu schaffen, die einfach herzustellen, durch eine relativ kleine Öffnung in der Bauchdecke in die Bauchhöhle eingebracht werden kann und darüber hinaus sichere Operationsbedingungen schafft.

Die der Erfindung zugrundeliegende Aufgabe wird durch die im Anspruch 1 angegebene Lehre gelöst.

Der Grundgedanke der Erfindung besteht darin, die schlauchförmigen Hohlkörper langgestreckt auszubilden, so daß sie nach Füllung mit einem Gas oder einer Flüssigkeit steife Stangen bilden, die sich mit ihren Enden an jeweils einem schlauchförmigen und einen Ring bildenden und über eine Schlauchleitung füllbaren Hohlkörper abstützen, derart, daß die langgestreckten Hohlkörper eine kegelstumpfförmige Wandung bilden, die in zusammengefaltetem Zustand durch eine kleine Öffnung in der Bauchdecke in die Bauchhöhle eingebracht und dort durch Füllung der Hohlkörper in eine steife Torroidform gebracht werden, so daß sie die Bauchdecke im Abstand zu einem Operationsort halten. Die Torroidform ist kegelstumpfförmig, wobei es zweckmäßig ist, den Rand der Kegelstumpfform mit dem kleineren Durchmesser auf dem Operationsfeld zu plazieren. Die Einrichtung ist daher einerseits in der Lage, die Bauchdecke ausreichend abzustützen, andererseits das Operationsfeld von seitlich hereindrängenden Organstrukturen, z. B. Darmschlingen, freizuhalten. Die Innenräume mehrerer oder aller Hohlkörper können miteinander verbunden sein, so daß nur eine Schlauchleitung zu ihrer Befüllung erforderlich ist oder wenige Schlauchleitungen sind.

Gemäß der Erfindung sind die Innenräume der langgestreckten Hohlkörper in Achsrichtung abwechselnd mit dem Hohlraum des einen und dem Hohlraum des anderen, einen Ring bildenden Hohlkörpers verbunden, wobei die ringförmigen Hohlkörper jeweils eigene Schlauchleitungen zur Füllung ihrer Hohlräume und der jeweils damit verbundenen Hohlräume der langgestreckten Körper aufweisen. Somit sind zwei Stützsysteme gebildet, die unabhängig voneinander sind, so daß bei Beschädigung eines der Hohlkörper beispielsweise durch ein Operationsinstrument immer noch eine ausreichende Abstützung der Bauchdecke gewährleistet ist.

Gemäß einer Weiterbildung der Erfindung sind die Hohlkörper durch zwei Kunststoffolien gebildet, die im Bereich der Begrenzungen der Hohlkörper miteinander verschweißt sind. Diese Kunststoffolien können sehr dünn sein, so daß sie im zusammengefalteten Zustand durch eine verhältnismäßig kleine Öffnung in der Bauchdecke in die Bauchhöhle eingebracht werden können, wonach sie durch pneumatische oder hydraulische Füllung zu steifen Körpern werden, die in der Lage sind, die Bauchdecke anzuheben und abzustützen und so das Operationsfeld freizuhalten.

Zweckmäßigerweise sind an einem oder beiden der jeweils einen Ring bildenden Hohlkörper Vorsprünge angeordnet, die mittels eines chirurgischen Fadens mit benachbarten Körperteilen, insbesondere mit der Bauchdecke, verbindbar sind, um so die Einrichtung zu fixieren. Diese Vorsprünge können die Form von einzelnen Laschen haben oder umlaufende Vorsprünge, z. B. überstehende Schweißkanten, bilden.

Anhand der Zeichnung soll die Erfindung an einem Ausführungsbeispiel näher erläutert werden.
- Fig. 1: zeigt das Ausführungsbeispiel in Seiten- ansicht und
- Fig. 2: zeigt das Ausführungsbeispiel gemäß Fig. 1 in perspektivischer Darstellung.

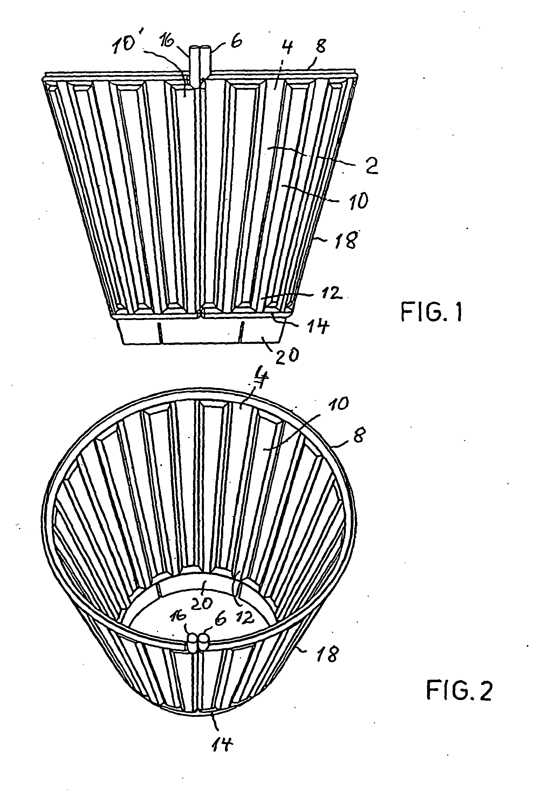

Die Einrichtung gemäß Fig. 1 weist langgestreckte, dünnwandige und zusammenfaltbare Hohlkörper 2 auf, die sich mit ihren oberen Enden jeweils an einem schlauchförmigen, dünnwandigen, einen Ring bildenden und über eine Schlauchleitung 6 füllbaren Hohlkörper 8 abstützen, wobei das Innere der schlauchförmigen Hohlkörper 2 und des schlauchförmigen Hohlkörpers 8 miteinander verbunden sind, so daß beide Hohlkörper über die Schlauchleitung 6 durch Gas oder eine Flüssigkeit füllbar sind.

Außerdem weist die Einrichtung in gleicher Weise wie die Hohlkörper 2 ausgebildete dünnwandige und zusammenfaltbare Hohlkörper 10 auf, die sich mit ihren unteren Enden 12 an einem dünnwandigen, zusammenfaltbaren, schlauchförmigen und einen Ring bildenden Hohlkörper 14 abstützen, wobei das Innere der schlauchförmigen Körper 10 mit dem Inneren des ringförmigen schlauchförmigen Hohlkörpers 14 jeweils verbunden ist. Das Innere einer der schlauchförmigen Körper 10, in der Zeichnung mit 10' bezeichnet, ist mit einer Schlauchleitung 16 verbunden, so daß über diese Schlauchleitung und das Innere des schlauchförmigen Körpers 10' auch das Innere des ringförmigen schlauchförmigen Körpers 14 mit Gas oder einer Flüssigkeit und damit auch das Innere sämtlicher damit verbundener schlauchförmiger Hohlkörper 10 füllbar ist.

Sämtliche langgestreckten schlauchförmigen Hohlkörper 2 und 10 sind quer zu ihrer Längsausdehnung miteinander verbunden. Der ringförmige Hohlkörper 8 hat einen größeren Durchmesser als der ringförmige Hohlkörper 14, so daß die miteinander verbundenen Hohlkörper 2 und 10 eine kegelstumpfförmige Wandung 18 bilden, die in einen steifen Zustand versetzbar ist, wenn sämtliche Hohlkörper 2, 10, 8 und 14 durch Einleitung beispielsweise einer Flüssigkeit in die Schlauchleitungen 6 und 16 prall gefüllt sind. Das bedeutet, daß in diesem Zustand beispielsweise der ringförmige Hohlkörper 8 eine Bauchdecke gegenüber einem inneren Körperteil im Abstand hält, an dem der ringförmige Hohlkörper 14 anliegt.

An dem unteren ringförmigen Hohlkörper 14 befinden sich Laschen 20, mit denen die gesamte Einrichtung durch einen oder mehrere chirurgische Fäden fixierbar ist.

Fig. 2 zeigt die Einrichtung gemäß Fig. 1 in perspektivischer Darstellung, so daß deutlich erkennbar ist, daß die Wandung 18 insgesamt einen kegeligen Hohlzylinder bildet.

Die Innenräume der langgestreckten Hohlkörper 2 und der Innenraum des ringförmigen Hohlkörpers 8 einerseits und die Innenräume der langgestreckten Hohlkörper 10 und des ringförmigen Hohlkörpers 14 andererseits bilden zwei getrennte Systeme, die jeweils getrennt über die Schlauchleitungen 6 bzw. 15 füllbar sind. Das bedeutet, daß bei Verletzung eines Hohlkörpers einer der beiden Gruppen die Hohlkörper der anderen Gruppe weiterhin die Stützwirkung entfalten können. Dadurch ist eine erhöhte Sicherheit während einer Operation gegeben.

## Patentansprüche

1. Einrichtung zum Abstützen der Bauchdecke gegenüber darunterliegenden Organen zum Freihalten eins Operationsraumes bei der minimalinvasiven Chirurgie, mit mehreren, über eine Schlauchleitung füllbaren schlauchförmigen und eine Torroidform bildenden, dünnwandigen und zusammenfaltbaren Hohlkörpern,
- wobei die schlauchförmigen Hohlkörper (2, 10) langgestreckt sind und sich in Richtung der Torroidform erstrecken,
- die schlauchförmigen Hohlkörper (2, 10) in Umfangsrichtung der Torroidform miteinander verbunden sind, und
- sich die langgestreckten Körper in Achsrichtung der Torroidform mit ihren Enden (4, 12) an jeweils einem schlauchförmigen, dünnwandigen, zusammenfaltbaren, einen Ring bildenden und über eine Schlauchleitung füllbaren Hohlkörper (8, 14) abstützen, die sich längs der beiden Ränder der Torroidform erstrecken, und
- die Durchmesser der beiden einen Ring bildenden Hohlkörper (8, 14) verschieden sind, derart, daß die langgestreckten Hohlkörper (2, 10) eine kegelstumpfförmige Wandung (18) bilden,
**dadurch gekennzeichnet,**
- **daß** die Innenräume der langgestreckten Hohlkörper (2, 10) in Achsrichtung abwechselnd mit dem Hohlraum des einen (8) und dem Hohlraum des anderen, einen Ring bildenden Hohlkörper (14) verbunden sind und
- **daß** die ringförmigen Hohlkörper (8, 14) jeweils eigene Schlauchleitungen (6, 16) zur Füllung ihrer Hohlräume und der jeweils damit vebundenen Hohlräume der langgestreckten Körper (2, 10) aufweisen.

2. Einrichtung nach Anspruch 1, **dadurch gekennzeichnet, daß** die Innenräume mehrerer oder aller Hohlkörper (2, 10, 8, 14) miteinander verbunden sind.

3. Einrichtung nach Anspruch 1, **dadurch gekennzeichnet, daß** die Hohlkörper (2, 10, 8, 14) durch zwei Kunststoffolien gebildet sind, die im Bereich der Begrenzungen der Hohlkörper (2, 10, 8, 14) miteinander verschweißt sind.

4. Einrichtung nach Anspruch 1, **dadurch gekennzeichnet, daß** an einem oder beiden der jeweils einen Ring bildenden Hohlkörper (14) Vorsprünge angeordnet sind, die mittels eines chirurgischen Fadens mit benachbarten Körperteilen, insbesondere mit der Bauchdecke, verbindbar sind, um die Einrichtung zu fixieren.

5. Einrichtung nach Anspruch 4, **dadurch gekennzeichnet, daß** die Vorsprünge durch einzelne Laschen (20) oder umlaufende Vorsprünge gebildet sind.

## Claims

1. Device for supporting the abdominal wall away from organs positioned underneath for freeing up a surgical space in minimally invasive surgery, comprising multiple tube-shaped, thin-walled, foldable hollow bodies forming a toroidal shape, which can be filled via a hose line;
- wherein the tube-shaped hollow bodies (2, 10) are elongated and extend in the direction of the toroidal shape;
- the tube-shaped hollow bodies (2, 10) are connected to each other in circumferential direction of the toroidal shape; and
- the elongated bodies are supported in an axial direction of the toroidal shape at their ends (4, 12) on a respective tube-shaped, thin-walled, foldable hollow body (8, 14), which forms a ring and a hollow body that can be filled via a hose line, which extend along both edges of the toroidal shape,
- the diameters of the two hollow bodies (8, 14) forming a ring are different, such that the elongated hollow bodies (2, 10) form a frustoconical wall (18),
**characterised in that**
- the interiors of the elongated hollow bodies (2, 10) are connected in axial direction to the hollow space of one hollow body (8), and, alternately, to the hollow space of the other hollow body (14) forming a ring; and
- **in that** the annular hollow bodies (8, 14) each have respective dedicated hose lines (6, 16) for filling their hollow spaces, and each have respective hollow spaces of the elongated bodies (2, 10) connected therewith.

2. Device according to claim 1, **characterised in that** the interiors of several or all of the hollow bodies (2, 10, 8, 14) are connected together.

3. Device according to claim 1, **characterised in that** the hollow bodies (2, 10, 8, 14) are formed by two plastic sheets, which are welded to each other in the region of the boundaries of the hollow bodies (2, 10, 8, 14).

4. Device according to claim 1, **characterised in that** protrusions are arranged on one or both of the hollow bodies (14) each forming a respective ring, which can be connected to adjacent body parts, particularly to the abdominal wall, by means of surgical threads, in order to secure the device.

5. Device according to claim 4, **characterised in that** the protrusions are formed by individual tabs (20) or by circumferential protrusions.

## Revendications

1. Dispositif de soutien de la paroi abdominale par rapport aux organes sous-jacents, destiné à dégager un espace chirurgical lors de la chirurgie minimalement invasive, comportant plusieurs corps creux de forme tubulaire à paroi mince, qui peuvent être remplis par l'intermédiaire d'une conduite tubulaire, forment un corps toroïdal et sont aptes à être repliés,
- sachant que les corps creux tubulaires (2, 10) sont allongés et s'étendent dans la direction du corps toroïdal,
- les corps creux tubulaires (2, 10) sont assemblés entre eux dans la direction circonférentielle du corps toroïdal, et
- les corps allongés prennent appui avec leurs extrémités (4, 12), dans la direction axiale du corps toroïdal, sur des corps creux (8, 14) tubulaires à paroi mince, repliables, lesquels forment un anneau et peuvent être remplis par l'intermédiaire d'une conduite tubulaire et lesquels s'étendent le long des deux bords du corps toroïdal, et
- les diamètres des deux corps creux (8, 14) formant un anneau sont différents, de telle sorte que les corps creux allongés (2, 10) forment une paroi (18) en forme de cône tronqué,
**caractérisé**
- **en ce que** les espaces intérieurs des corps creux allongés (2, 10) sont reliés dans la direction axiale en alternance avec la cavité de l'un des corps creux en forme d'anneau (8) et avec la cavité de l'autre corps creux en forme d'anneau (14), et
- **en ce que** les corps creux en forme d'anneau (8, 14) comportent chacun leur propre conduite tubulaire (6, 16) pour le remplissage de leur cavité et les cavités, reliées respectivement à cette dernière, des corps creux allongés (2, 10).

2. Dispositif selon la revendication 1, **caractérisé en ce que** les espaces intérieurs de plusieurs ou de tous les corps creux (2, 10, 8, 14) sont reliés entre eux.

3. Dispositif selon la revendication 1, **caractérisé en ce que** les corps creux (2, 10, 8, 14) sont formés par deux feuilles plastiques, qui sont soudées l'une à l'autre dans la zone des délimitations des corps creux (2, 10, 8, 14).

4. Dispositif selon la revendication 1, **caractérisé en ce que** sur l'un ou sur les deux corps creux (14) formant chacun un anneau sont réalisées des saillies, qui peuvent être assemblées au moyen d'un fil chirurgical à des parties du corps adjacentes, en particulier à la paroi abdominale, afin d'immobiliser le dispositif.

5. Dispositif selon la revendication 4, **caractérisé en ce que** les saillies sont formées par des pattes (20) individuelles ou par des saillies périphériques.
